# EUROPEAN PATENT APPLICATION

(11) **EP 1 479 690 A1**
(43) Date of publication of application: **24.11.2004**
(21) Application number: 04012244.2
(22) Date of filing: 24.05.2004
(51) Int. Cl.: C07K 7/08, C12N 15/11

(54) **Cytokine isolated from an insect**

(30) Priority: 23.05.2003 JP 2003146551
(71) Applicant: National University Corporation Hokkaido University, Sapporo 060-0808 (JP)
(72) Inventor: Hayakawa, Yoichi, Sapporo-shi Hokkaido (JP)
(74) Representative: Heusler, Wolfgang, Dipl.-Ing.

(57) **Abstract**

A peptide for inducing proliferation of an insect cell, which consists of an amino acid sequence (Thr-Ile-Leu-Ser-Ala-Pro-Ser-Asn-Cys-Gln-Gln-Thr-Asp-Phe-Lys-Gly-Arg-Cys-Leu).

SEQUENCE LISTING

<110>  President of Hokkaido University

<120>  A novel cytokine isolated from an insect

<130>  03S1637-2

<140>  EP

<150>  JP 2003-146551

<160>  3

<170>  PatentIn version 3.1

<210>  1
<211>  19
<212>  PRT
<213>  Lucilia cuprina

<400>  1

Thr Ile Leu Ser Ala Pro Ser Asn Cys Gln Gln Thr Asp Phe Lys Gly
1               5                   10                  15

Arg Cys Leu

<210>  2
<211>  57
<212>  DNA
<213>  Lucilia cuprina

<220>
<221>  CDS
<222>  (1)..(57)
<223>

<400>  2
acg att tta agc gct cct tcc aat tgt caa caa aca gat ttt aaa ggt        48
Thr Ile Leu Ser Ala Pro Ser Asn Cys Gln Gln Thr Asp Phe Lys Gly
1               5                   10                  15

cgt tgt tta                                                            57
Arg Cys Leu

<210>  3
<211>  19
<212>  PRT
<213>  Lucilia cuprina

<400>  3

Thr Ile Leu Ser Ala Pro Ser Asn Cys Gln Gln Thr Asp Phe Lys Gly
1               5                   10                  15

Arg Cys Leu

## Description

The present invention relates to a novel cytokine of an insect.

Cytokines are proteinous hormone-like substances produced by a variety of organisms and regulating cellular functions such as proliferation, differentiation, and expression of cell function. In general, a cytokine acts specifically on cells having receptors of the cytokine expressed thereon. Since first discover of a growth factor such as an epidermal growth factor (EGF), a wide variety of cytokines have been found principally in mammalians. At present, cytokines have been received attention as bioactive substances in various fields from basic to applied research including regenerative medical techniques. Actually, some of them are practically used in clinical treatments such as cancer treatment and skin transplantation.

Cytokines derived from insects include a growth-blocking peptide (GBP), paralytic peptide (PP), and plasmatocyte-spreading peptide (PSP). Since these peptides have a common sequence of Gln(E)-Asn(N)-Phe(F) at the amino terminal, they are provisionally called "ENF peptides". These ENF peptides, up to now, have been identified only in lepidopteran insects.

In consideration of these circumstances, it is an object of the present invention to provide a novel insect cytokine, and more specifically, a novel cytokine useful for inducing proliferation of insect cells.

More specifically, the present invention provides following peptides:
A peptide for inducing a cellular response from cells, which consists of an amino acid sequence selected from the group consisting of:
   a) an amino acid sequence represented by Sequence ID No. 1; and
   b) an amino acid sequence represented by Sequence ID No. 1, except that one or several amino acids are deleted, replaced or added.

Hereinafter, a peptide provided by the present invention is generally referred to as a "peptide according to the present invention".

This summary of the invention does not necessarily describe all necessary features so that the invention may also be a sub-combination of these described features.

The invention can be more fully understood from the following detailed description when taken in conjunction with the accompanying drawings, in which:
FIG. 1 shows an amino acid sequence of a peptide according to the present invention;
FIGS. 2A and 2B are photomicrographs of hemocytes of an L. cuprina larva treated with a peptide according to the present invention and bovine serum albumin (BSA, (control)), respectively; and
FIG. 3 is a graph showing [³H] thymidine incorporation into a cell strain, High five, treated with a peptide according to the present invention and BSA (control).

The present invention will be described in detail below.

The inventor of the present invention have succeeded in isolating a peptide according to the present invention, i.e. by purifying the peptide based on a bioactive function (more specifically, hemotocyte aggregation reaction) of the peptide for hemocytes (i.e., hemolymph cells) of the last instar fly larvae, Lucilia cuprina. The bioassay method using insect hemocytes according to the present invention was a novel method carried out for the first time by the present inventor. This method has a high sensitivity and reproducibility. Therefore, even though a small living organism such as a fly larva was used, a peptide according to the present invention was identified and purified.

The present inventor has obtained cDNA of a gene encoding a peptide according to the present invention. The cDNA was obtained by a known procedure based on a partial amino acid sequence of a purified peptide according to the present invention. To explain this procedure more specifically, first, a partial amino acid sequence of the purified peptide is determined. Then, various primers are prepared based on the amino acid sequence. Subsequently, using these primers, PCR cloning is performed to amplify a nucleic acid fragment encoding the gene. Thereafter, using the nucleic acid fragment as a probe, cDNA library prepared from hemocytes of a fly (L. cuprina) is screened. In this manner, cDNA is cloned. The cloned cDNA has a length of 555 base pairs and its open reading frame encodes a pre-pro-peptide of 112 amino acid residues. A peptide according to the present invention has an amino acid sequence positioned at the C terminal region of the pre-pro-peptide.

### <Features of peptide according to the present invention>

A peptide according to the present invention has the lowest molecular weight (19 amino acid residues) of various cytokines ever known. For this reason, a peptide according to the present invention may be defined as a peptide cytokine. The peptide composed of 19 amino acid residues can be chemically synthesized. The lowermost weight mammalian cytokine ever found is an epithelial growth factor (EGF). Even if the smallest one, the EGF has about 50 residues. However, it is impossible to synthesize chemically an about 50-residue peptide in a large amount by conventional technique.

As described, a peptide according to the present invention is a cytokine for the first time obtained by overcoming technical difficulties.

### <Outline of the Present Invention>

According to the present invention, there is provided a peptide capable of inducing a cellular response from cells, comprising an amino acid sequence represented by Sequence ID No. 1 where one or several amino acids may be deleted, replaced or added. Accordingly, examples of the peptide include a peptide constituted of an amino acid sequence represented by Sequence ID No. 1 and other peptides having the same activity.

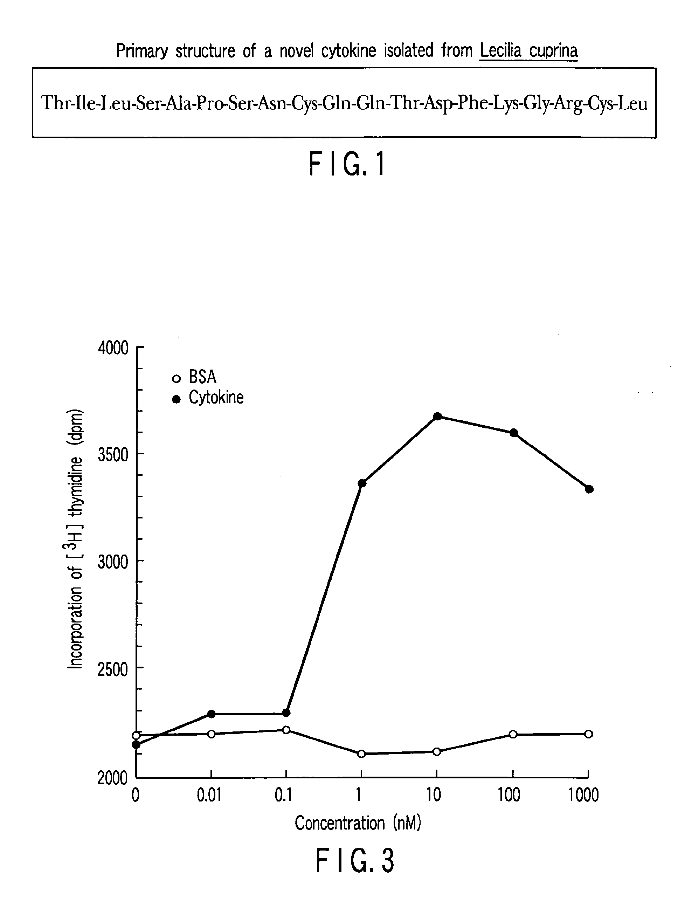

The present invention also provides a nucleic acid sequence encoding a peptide according to the present invention. Furthermore, the present invention provides a recombinant vector containing the nucleic acid sequence. Moreover, the present invention provides a transformant containing the vector. The techniques for obtaining a nucleic acid sequence based on an amino acid sequence of a peptide, constructing a recombinant vector and a transformant and then using them are known to those skilled in the art. The amino acid sequence represented by Sequence ID No. 1 consists of 19 residues: 
Thr-Ile-Leu-Ser-Ala-Pro-Ser-Asn-Cys-Gln-Gln-Thr-
Asp-Phe-Lys-Gly-Arg-Cys-Leu (sequentially from the N
terminal);
 as is shown in FIG. 1.

The term "cellular response" refers to a physical response generated from cells when a peptide according to the present invention is applied the cells. The cellular response may vary depending upon the type of cells to which a peptide according to the present invention is applied. Examples of the cellular response include proliferation, differentiation, migration and generation of anti-bacterial agent.

The expression of "inducing a cellular response" refers to inducing a cellular response de-novo or enhancing the cellular response already induced.

A preferable cellular response induced by a peptide according to the present invention includes "hemocyte aggregation" and "cell proliferation".

Now, the "hemocyte aggregation" and "cell proliferation" will be explained below.

### [Hemocyte aggregation]

As previously explained, a peptide according to the present invention is a physiologically active peptide purified based on a physiological activity of inducing aggregation of hemocytes of a fly (Lucilia cuprina). The hemocyte aggregation may result from increased adhesiveness of cells by the action of a peptide according to the present invention. Accordingly, at least one of the actions of a peptide according to the present invention may be involved in activating a signal transduction pathway leading to cell adhesion.

The activation of the signal transduction pathway can be detected by observing changes of the following factors before and after addition of a peptide according to the present invention.
(a) Formation of cell aggregation: cells are adhered to each other to form an aggregate when a peptide according to the present invention acts upon the cells;
(b) Adhesion to other substance: when a peptide according to the present invention acts upon cells, the adhesiveness of cells to other substances besides the cells is increased. Examples of other substances include non-biological substances such as a culture plate; biological substances such as pathogens (e.g. bacteria); and substances such as proteins derived from the biological substances.

The hemocytes are responsible for mediating cellular immunity and thus correspond to mammalian leukocytes, which attack phatogens. The leukocytes are usually circulating within the body without adhering to the blood wall. However, when a pathogen invades, the leukocytes adhere to the blood wall and further migrate into the tissue and reach the infection site into which the pathogen invades. The leukocytes such as phagocytes adhere to the pathogen to kill it. The cellular response of the leukocytes in increasing adhesiveness of cells for mediating their function is analogous to that of the hemocytes. This analogy suggests that the hemocyte aggregation induced by a peptide according to the present invention may play a role in immune response in an insect.

### [Cell proliferation]

The term "cell proliferation" refers to one of cellular responses produced when a peptide according to the present invention is applied to cells. More specifically, the "cell proliferation" refers to increasing the number of cells by cell division induced by a peptide according to the present invention, compared to the number of cells to which a peptide according to the present invention is not applied. The cell division is mediated through the signal transduction pathway leading to DNA replication and cell division. Therefore, the concept "cell proliferation" includes an increase of the amount of intracellular DNA resulted from DNA replication which is caused when a peptide of the present invention acts upon cells.

### Target cells:

Target cells of a peptide according to the present invention are not particularly limited as long as the cells are proliferated when the peptide is applied thereto. The origin from which the target cells are derived is not particularly limited. More specifically, the origin of the target cells may be a mammal, preferably cultured insect cells, more preferably, cultured cells of an lepidopteran insect, most preferably, High five, which is a cell strain derived from the oocyte cell of Trichoplusia ni.

### Culture conditions:

The culturing conditions may be the same as conventional culture conditions usually employed for culturing cells except that a peptide according to the present invention is added to a culture medium in place of the serum. The concentration of a peptide according to the present invention may be set depending upon conditions and with reference to the results of a preliminary experiment carried out at various concentrations. The concentration is generally within the range of ~1,000 nM, preferably 0.1 to 1,000 nM, more preferably 1 to 1,000 nM, and most preferably 10 to 100 nM.

The High five cells may be proliferated in vitro, for example, in the following conditions:
Culture solution: Grace's medium (manufactured by GIBCO, USA)
Peptide concentration: 10 to 100 nM
Temperature: 25°C

### Applications:

The technique for culturing insect cells to which a peptide according to the present invention is to be applied has been used in the field where medicaments and insecticides are developed. For example, the insect cells cultured are used for producing a useful substance therein by use of the expression system of Baculovirus and for determining the sensitivity of the cell to a drug such as an insecticide.

### <Preparation of peptide according to the present invention>

A peptide according to the present invention can be produced by a known chemical synthetic method of a peptide, such as t-Boc method or F-moc method. Alternatively, the peptide may be synthesized by ordering synthesis of the peptide to a chemical synthesis company. Moreover, the peptide may be obtained by purifying it from hemocytes of a fly (Lucilica cuprina) based on the aggregation activity of the hemocytes described in "hemocyte aggregation assay" set forth in Examples below. The peptide can be purified by using a known method of purifying a protein and peptide, for example, in accordance with the "preparation of peptide" described in the Examples below.

The present invention will be explained in detail by way of Examples, which should not be construed as limiting the scope of the present invention.

### [Examples]

### <Preparation of peptide>

### [Insect]

Flies (Lucilia cuprina) were reared at 25°C by feeding them with the porcine liver under the optical conditions where a 16-hour bright period and an 8-hour dark period were alternately changed.

### [Purification method]

The whole blood (hemolymph) was taken from the 3rd instar larvae of the flies (24 to 36 hours after hatching) directly into ice-cold 50% acetone (1 ml). Immediately after the hemolymph was diluted, about 1.5 ml of the diluted hemolymph was subjected to centrifugation at 20,000 g at 4°C for 10 minutes. The supernatant was dried by Speed-Vac to obtain a crude peptide.

The crude peptide was redissolved in 0.05% trifluoroacetic acid. This was subjected to a reverse-phase C18 HPLC column (Capcell Pak C18UG 120; 4.6 × 150 mm, manufactured by Shiseido Co., Ltd. , Japan) and separated by gradient elution of 0 to 50% of CH₃CN contained in a 0.05% aqueous solution of trifluoroacetic acid at a flow rate of 1 ml/minute. Next, bioactive fractions exhibiting an activity of aggregating hemolymph were concentrated by Speed-Vac and filtrated through a filter (Ultrafree MC 5,000 manufactured by Milipore Corporation, USA), thereby cut off a substance of 5000 daltons or more. The filtrate was subjected to a reverse-phase C4 HPLC column (Mightysil RP-4 GP; 4.6 × 250 mm, Kanto Chemical Co., Ltd., Japan) and separated by gradient elution of 5 to 25% of CH₃CN contained in a 0.05% aqueous solution of trifluoroacetic acid at a flow rate of 1 ml/minute. The bioactive fractions were subjected to a reverse-phase C18 HPLC column (Mightysil RP-18 GP; 4.6 × 250 mm, Kanto Chemical Co., Ltd., Japan) and separated by gradient elution of 10 to 40% of CH₃CN contained in a 0.05% aqueous solution of trifluoroacetic acid at a flow rate of 0.5 ml/minute. Bioactive fractions were subjected to a reverse-phase C4 HPLC column (Protein-RP; 4.6 × 250 mm, manufactured by YMC , Japan) and separated by gradient elution of 10 to 40% of CH₃CN contained in an aqueous 0.05% trifluoroacetic acid at a flow rate of 0.5 ml/minute. In the final purification step, the bioactive fractions were subjected to the lineally connected reverse-phase C18 HPLC columns (Mightysil RP-18 GP; 4.6 × 250 mm, Kanto Chemical Co., Ltd., Japan), and separated by gradient elution of 18 to 33% of CH₃CN contained in a 0.05% trifluoroacetic acid at a flow rate of 0.5 ml/minute. The bioactive fractions were stored.

A peptide according to the present invention is purified in the manner mentioned above and stored in accordance with a known proper storage method until use.

### <Preparation of hemolymph for use in functional analysis>

Blood (hemolymph) was taken from the 3rd instar flies (Lucilia cuprina) after 24 to 36 hours from molting in an anti-coagulant solution (98 mM NaOH, 186 mM NaCl, 17 mM Na₂ EDTA and 41 mM citric acid, pH 4.5). Hemolymph was obtained as sediment by centrifuging at 400g for one minute, suspended in the anti-coagulation solution, incubated at 4°C for 30 minutes, and subjected to centrifugal separation at 400 g for one minute to collect hemolymph. The hemolymph thus collected was resuspended in Ex-cell 400 medium (manufactured by JRH bioscience, USA)

### <Analysis for peptide function>

The activity of the peptide was investigated as follows:

### [Hemocyte aggregation assay]

### Methods:

Using hemolymph prepared above, an ability of a peptide according to the present invention for aggregating hemocytes was evaluated. 5 µl of a test substance (so as to obtain a final concentration of 10 nM) and 30 µl of a blood cell suspension solution were delivered to 96 wells of a culture plate so as to obtain a cell concentration of 1 × 10⁴ cells/well. As the test substance, a peptide according to the present invention (a final concentration of 10 nM) or bovine serum albumin (a final concentration of 10 nM) as a control was used. The mixture was observed by an inverted phase-contrast microscope (Olympus IX-70) to detect hemocyte aggregation. When a mixture of the test substance with hemocytes was incubated for one hour, if hemocyte aggregation was not observed, it was determined that the test substance does not have a function for aggregating hemocytes.

### Results:

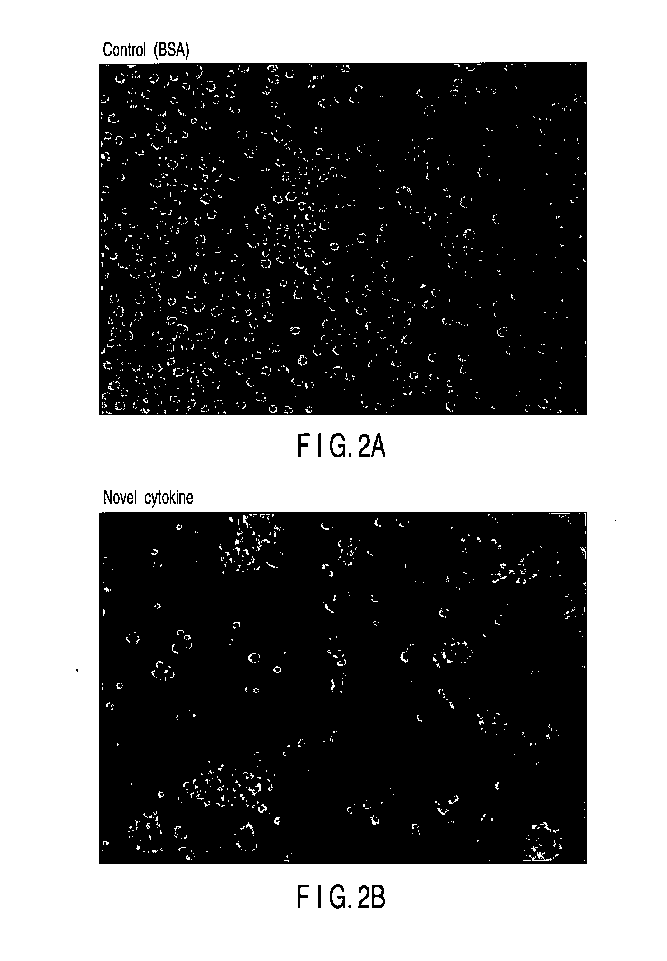

FIG. 2B shows a microscopic image of hemocytes treated with 10 nM of a peptide according to the present invention. The hemocytes were isolated from a fly (L. Cuprina) and mixed with 10 nM of said peptide and then incubated for about 20 minutes. FIG. 2A also shows the result of the control hemocytes treated with bovine serum albumin (BSA) in the same amount as said peptide. In the control, it is found that the hemocytes are scattered without causing aggregation, whereas hemocyte aggregation is observed in cells to which a peptide according to the present invention is used as a cytokine.

### [Cell proliferation assay]

### Methods:

Cultured insect cells of "High five" was purchased from Invitrogen (Holland) and maintained in Grace's medium containing 5% fetus bovine serum. The cells during the exponential growth period were used in the following treatment.

After the cells were cultured in a culture solution containing only Grace's medium (with no fetus bovine serum) for 2 days, they were divided into two groups. To one group (novel cytokine group), a peptide according to the present invention was added as a test substance in an amount of ~ 1,000 nM. To the other group (control group), bovine serum albumin (BSA) was added as a test substance in an amount of ~ 1000 nM. Both groups were cultured for 32 hours in Grace's medium containing 0.5 µCi of [³H] thymidine. Subsequently, Both groups of cells were washed three times with a phosphate buffered saline (8 mM Na₂HPO₄, 1.5 mM KH₂PO₄, 137 mM NaCl, 2.7 mM KCL, pH 7.2). Thereafter, 100 µl of 0.3 N NaOH was added to both groups of cells to lyse the cells. The lysed cells were subjected to the liquid scintillation counter (Aloka LSC-5100 manufactured by Aloka, Japan). In this way, the radioactive count of [³H] thymidine, which had been incorporated in the cells of each group during culturing, was determined.

### Results:

In a novel cytokine group where a peptide according to the present invention was applied to the High five cells, [³H] thymidine incorporation was increased in a pepetide-concentration dependent manner (see FIG. 3). However, in the control group treated with BSA, [³H] thymidine incorporation was not increased (FIG. 3).

## Claims

1. A peptide consisting of an amino acid sequence represented by Sequence ID No. 1.

2. A peptide for inducing proliferation of an insect cell, which consists of an amino acid sequence selected from the group consisting of:
a) an amino acid sequence represented by Sequence ID No. 1; and
b) an amino acid sequence represented by Sequence ID No. 1, except that one or several amino acids are deleted, replaced or added.

3. The peptide according to claim 2, **characterized in that** the insect cell is derived from an insect belonging to Lepidoptera.

4. The peptide according to claim 2, **characterized in that** the insect cell is High five.

5. A nucleic acid encoding the peptide according to any one of claims 1 to 4.

6. A recombinant vector containing the nucleic acid according to claim 5.

7. A transformant containing the recombinant vector according to claim 6.
